(19) Europäisches Patentamt
European Patent Office
Office européen des brevets

(11) EP 2 401 346 B1

(12) **EUROPEAN PATENT SPECIFICATION**

(45) Date of publication and mention of the grant of the patent:
**22.01.2014 Bulletin 2014/04**

(21) Application number: **09785736.1**

(22) Date of filing: **08.07.2009**

(51) Int Cl.:
***C10L 1/224** (2006.01)* ***C07D 233/08** (2006.01)*

(86) International application number:
**PCT/GR2009/000047**

(87) International publication number:
**WO 2010/097640 (02.09.2010 Gazette 2010/35)**

(54) **BIODIESEL CONTAINING NON-PHENOLIC ADDITIVES AND THEREBY POSSESSING ENHANCED OXIDATIVE STABILITY AND LOW ACID NUMBER**

NICHTPHENOLISCHE ZUSATZSTOFFE ENTHALTENDER BIODIESEL, DER DADURCH EINE VERBESSERTE OXIDATIONSSTABILITÄT UND EINE NIEDRIGE SÄUREZAHL AUFWEIST

BIODIESEL CONTENANT DES ADDITIFS NON PHÉNOLIQUES ET POSSÉDANT DE CE FAIT UNE STABILITÉ À L'OXYDATION AMÉLIORÉE ET UN FAIBLE INDICE D'ACIDITÉ

(84) Designated Contracting States:
**AT BE BG CH CY CZ DE DK EE ES FI FR GB GR HR HU IE IS IT LI LT LU LV MC MK MT NL NO PL PT RO SE SI SK SM TR**
Designated Extension States:
**BA RS**

(30) Priority: **26.02.2009 GR 20090100113**

(43) Date of publication of application:
**04.01.2012 Bulletin 2012/01**

(73) Proprietor: **Dorivale Holdings Limited**
**P.C 1090 Nicosia (CY)**

(72) Inventors:
- **STOURNAS, Stamoulis**
  **GR-114 75 Athens (GR)**
- **KARAVALAKIS, Georgios**
  **GR-176 72 Kallithea Attiki (GR)**

(74) Representative: **Patentanwälte**
**Ruff, Wilhelm, Beier, Dauster & Partner**
**Postfach 10 40 36**
**70035 Stuttgart (DE)**

(56) References cited:
**EP-A- 1 847 583**
**WO-A-94/19430**
**WO-A-2008/049822**
**US-A1- 2008 282 605**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

EP 2 401 346 B1

## Description

FIELD OF THE INVENTION

This invention relates to improved biodiesel fuel. It is more particularly concerned with biodiesel fuel containing non-phenolic additives that enhance the oxidative stability and simultaneously decrease the acid number.

BACKGROUND OF THE INVENTION

Carbon dioxide emissions from fuel usage are generally considered a major contributor to global warming. As a means to alleviate the problem, an increased use of biomass derived fuel components (biofuels) in gasoline and diesel fuel is being pursued in many parts of the world. A significant portion of the total amount of biofuels used in the transportation sector is taken by biodiesel, whose consumption in the European Union is already in the millions of tons. The accepted source of biodiesel are various types of vegetable or animal fats and oils, which are generally transesterified to a mixture of fatty acid methyl or ethyl esters so as to reduce viscosity and improve combustion behavior. The resulting biodiesel is quite similar to conventional diesel fuel in many of its properties, such as viscosity, density and ignition quality. Biodiesel is also very compatible with conventional diesel and the two can be blended in any proportion.

In order for biodiesel to be acceptable as a fuel for diesel engines, either alone or mixed with conventional petrodiesel, it has to meet a number of quality criteria irrespective of the raw material that was used for its production. To this end, a number of specifications have been developed, such as EN 14214 in Europe and ASTM D-6751 in the United States, which strictly control the properties and characteristics of biodiesel fuels. The difference in chemical structure between biodiesel (a mixture of liquid carboxylic esters) and the conventional, petroleum derived diesel fuel or petrodiesel (a mixture of liquid hydrocarbons) leads to some notable differences in chemical behavior. Among the most important such differences are the lower stability of biodiesel towards oxidation and also its higher acidity in comparison to petrodiesel. Recognizing this, the European Standard EN 14214 places strict limits both as to the oxidative stability and as to the acidity of biodiesel. It is also noteworthy that the newest version of the European Standard EN 590 for road diesel, which allows the addition of up to 7% of biodiesel, also mandates that the final fuel blend meet specific new requirements as to its oxidative stability.

DESCRIPTION OF THE PRIOR ART

The oxidative stability of biodiesel has been the subject of extensive research in recent years. The consensus is that unsaturation in the fatty acid alkyl chain, particularly as it appears in polyunsaturated fatty acids, is the major cause of oxidative instability. One remedy for this situation is the careful selection of the raw materials used for biodiesel production so as to minimize the content of unsaturated fatty acids. The disadvantage of this approach is that large quantities of vegetable oils would be labeled as unfit for biodiesel production in the face of a constantly increasing demand for this renewable fuel. The alternative solution that has been widely adopted is the addition of small quantities of antioxidant additives that greatly enhance the oxidative stability of biodiesel. The vast majority of antioxidant additives that are used for this purpose are of the phenolic type, so named because they possess at least one hydroxyl group directly attached to an aromatic ring. Among the most common phenolic antioxidants are 2,6-di-tert-butyl-4-cresol, commonly known as BHT (butylated hydroxytoluene), 2,6-di-tert-butyl-4-methoxyphenol, commonly known as BHA (butylated hydroxyanisol), propyl gallate, 1,2,3-trihydroxybenzene (pyrogallol), and tert-butyl-hydroquinone (TBHQ). The chemical structures of these materials are drawn below, plainly displaying their common feature of one or more phenolic hydroxyls.

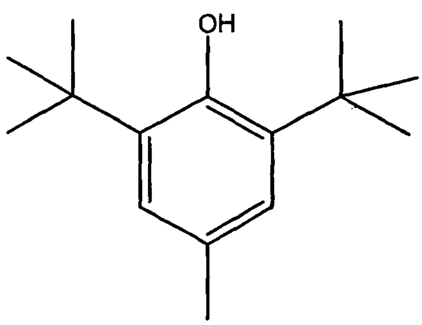

BHT

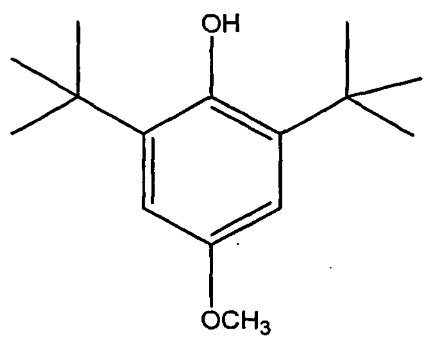

BHA

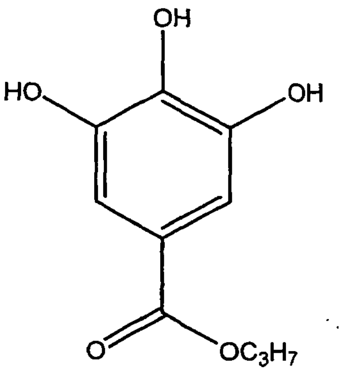

Propyl Gallate

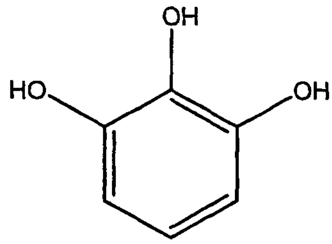

Pyrogallol

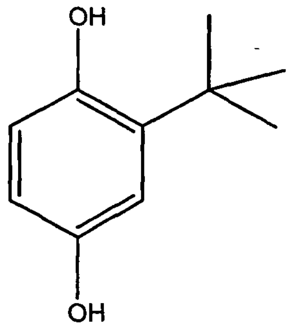

TBHQ

These and many other phenolic antioxidants have appeared in the technical literature (cf. Gerhard Knothe, "Some aspects of biodiesel oxidative stability", Fuel Processing Technology, vol. 88, pp. 669-677, 2007; Southwest Research Institute, "Characterization of biodiesel oxidation and oxidation products", CRC Project No. AVFL-2b, 2005; and references therein) and are also the subjects of various patents (e.g.) German Patent DE 10 252 714 A1, 2002; German Patent DE 10 2005 015 474 A1, 2006; European Patent EP 1989275, 2007).

References to other types of antioxidant additives for biodiesel are scant; a recent world patent (WO 2008 121526 A1) discloses antioxidant blends of aromatic diamines with phenolic antioxidants for biodiesel.

Phenolic antioxidants are generally effective in improving the oxidative stability of biodiesel. However, because of the acidic character of the phenolic hydroxyl, they tend to have a negative impact on the acid number of biodiesel. This behaviour was reported in a recent study, where it was observed that phenolic antioxidants of various types generally increased the acid number of biodiesel and sometimes brought it above the specification limit, thus making the biodiesel unfit for purpose (Sigurd Schober and Martin Mittelbach, "The impact of antioxidants on biodiesel oxidation stability", Eur. J. Lipid Sci. Technol., vol. 106, pp. 382-389, 2004).

The acid number is a measure of the amount of acidic components that exist in biodiesel or any other fuel. Since the acidic components are unwanted (they can lead to corrosion of the fuel system and general instability of the fuel itself) the acid number of biodiesel is strictly controlled both in Europe (EN 14214) and the United States (ASTM D-6751). Up to now no additives have been mentioned that would act as acid number depressants.

WO 94/19430 A discloses petroleum fuel antifoams which are used to control foaming in blends of petroleum-based fuel oil with biofuel. The corresponding antifoams can be specific reaction products obtainable by the reaction between a polyamine having at least one primary or secondary amino group and a carboxylic acylating agent. There is no hint in this document to use these antifoams for other purposes than to control foaming.

In WO 2008/049822 A use of specific oligo- or polyamines for increasing the oxidation stability of biofuel oils is shown. In this context, a limited number of amines is used in the examples of this document.

From the above considerations it becomes obvious that there is a need for antioxidant additives for biodiesel that will possess the same or higher antioxidant activity as the phenolic antioxidants without their negative impact on the acid number. The present invention offers an answer to this need.

## DESCRIPTION OF THE INVENTION

The basis of the invention is the discovery that the reaction product of a carboxylic acid and a polyamine, when added to biodiesel in small amounts, is capable of greatly improving the oxidative stability of the biodiesel and simultaneously lowering its acidity, as determined by measuring the acid number.

It is known in the art that when a carboxylic acid (I) is reacted with a 1,2-diamine (II) a 5-membered heterocyclic ring, imidazoline (III) is eventually formed by loss of water, as shown by reaction scheme (1):

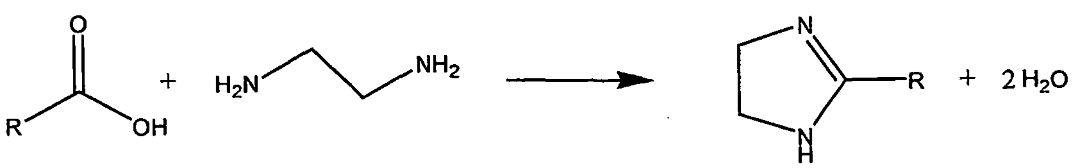

(1)

(I) (II) (III)

In a similar fashion, if a 1,3-diamine (IV) is reacted with a carboxylic acid, a 6-membered heterocyclic ring, tetrahydropyrimidine (V) is formed, as in reaction scheme (2):

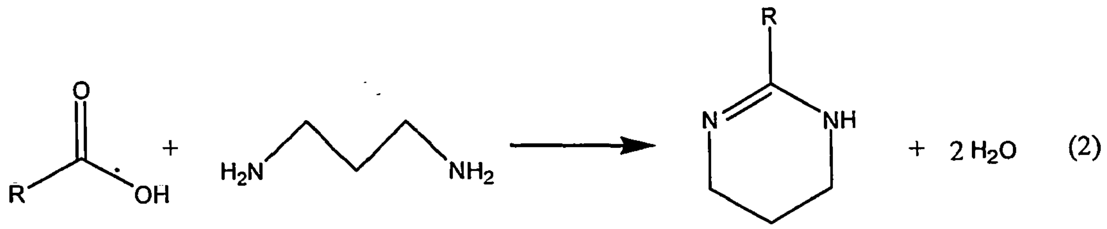

(I) (IV) (V)

As mentioned above, the unexpected discovery was made that certain preparations containing the imidazoline (formal name: 4,5-dihydro-1H-imidazole) or tetrahydropyrimidine (formal name: 1,4,5,6-tetrahydro-pyrimidine) moieties (III) and (V) are very effective antioxidants and acid number reducers for biodiesel and its mixtures with petrodiesel. Imidazoline and tetrahydropyrimidine derivatives have been mentioned in the patent literature as components in pharmaceutical preparations (e.g. U.S. patents 5,470,856; 5,925,665; 6,294,566; 6,410,562; 6,875,788; 6,884,801; 7,173,044; 7,482,358), corrosion inhibitors (e.g. U.S. patents 7,057,050;4,518,782), fuel detergents (e.g. U.S. patents 4,247,300; 2,961,308) etc. To our knowledge the use of imidazoline and tetrahydropyrimidine derivatives as additives that improve oxidation stability and lower acid number for biodiesel and biodiesel/petrodiesel blends has not been reported up to know.

For the purposes of this invention, the carboxylic acid portion in reactions (1) and (2) can be either a free acid or a lower ester (preferably a methyl or ethyl ester); in the latter case one mole of water and one mole of alcohol are evolved with the formation of the heterocyclic ring. The desired structure of the carboxylic moiety for the above reactions is shown in formula (VI):

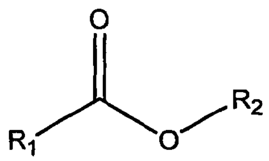

(VI)

Where:

- $R_1$ is a straight or branched alkyl or hydroxyalkyl group having the formula $C_mH_{2m-x-k+1}(OH)_k$ (m being an integer from 1 to 30, x being 0, 2, 4, or 6 but always fulfilling the relation x<m, and k being 0 or 1), a cyclohexyl group, a phenyl group, or a substituted phenyl group bearing a $C_1$ to $C_{12}$ alkyl substituent.
- $R_2$ is hydrogen, methyl, or ethyl.

Based on formula (VI), the carboxylic moiety can be, but is not limited to, any linear monocarboxylic acid from acetic to melissic; a branched carboxylic acid such as isovaleric or isooctanoic; an unsaturated carboxylic acid such as oleic, linoleic, or linolenic; a hydroxyacid such as ricinoleic; a carbocyclic acid such as cyclohexanoic; an aromatic acid such as benzoic or toluic; and the like. The methyl or ethyl esters of the above acids are also desirable. Mixtures of acids and/or esters, such as those derived from fats and oils by saponification or transesterification are particularly desirable because of their low cost.

The amine portion in reactions (1) and (2) must fulfill certain structural requirements. It must contain at least one primary amine nitrogen atom which is separated by two or three carbon atoms from a second amine nitrogen atom that is primary or secondary. More amine nitrogen atoms may be present and are desirable. Aromatic amines are not preferred because of their high toxicity. Thus the desired structure of the amine moiety is as shown in formula (VII):

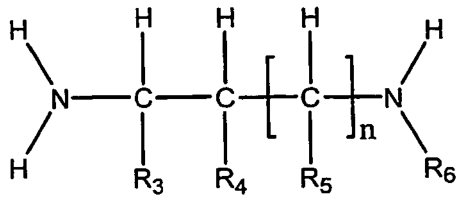

(VII)

Where:

- n is 0 or 1.
- $R_3$ and $R_4$ are hydrogen, methyl, or taken together are part of a cyclohexane ring.
- $R_5$ is hydrogen, or methyl.
- $R_6$ is hydrogen, a straight or branched alkyl group having the formula $C_mH_{2m-x+1}$ (m being an integer from 1 to 22 and x being 0, 2, 4, or 6 but always fulfilling the relation x<m), or an amine moiety having the formula $-CH_2CH_2(CH_2)_pNH[CH_2CH_2(CH_2)_pNH]_qH$ with p being 0 or 1 and q being 0, 1, 2, 3, or 4.

Based on formula (VII), the amine moiety can be a diamine, such as 1,2-diaminoethane, 1,2-diaminopropane, 1,3-diaminopropane, 1,2-diaminocyclohexane, and the like; commercially available mixtures of diamines, such as the various substituted 1,3-propanediamines with the trade name DUOMEEN, can also serve as the amine moiety. Polyamines, such as diethylene triamine (DETA), triethylene tetramine (TETA), tetraethylene pentamine (TEPA), dipropylene triamine, and the like are also desirable forms of the amine moiety. The commercially available polyamines such as TETA, TEPA, and their higher homologues are not pure compounds but mixtures of linear, branched, and cyclic polyamines. It is evident that the reaction products of these mixed polyamines with the carboxylic moieties of formula (VI) are not in their turn pure imidazolines or tetrahydropyrimidines; they were found, nevertheless, to be very effective antioxidants and acid number reducers for biodiesel and are therefore among the preferred embodiments of the present invention.

All the reaction products that are useful for the purposes of this invention, as described above, are obtained as solids or viscous liquids. In many instances it is convenient for handling purposes to employ them in the form of concentrated solutions. These solutions may contain from 5% to 95% by weight of the active ingredient dissolved in a suitable solvent, such as a liquid hydrocarbon mixture of high flash point, a biodiesel suitable for use in a compression ignition engine, or a mixture of the two.

The common structural feature of all the products arising from the reaction between a carboxylic acid (VI) and an amine (VII) is that of the imine group (VIII):

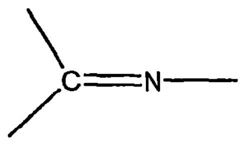

(VIII)

However, the imine group by itself is not sufficient to impart antioxidant activity. It was found that an open chain imine such as that in tetramethylguanidine (IX) is totally ineffective. Similarly, an imine such as that in an oxazoline (X) is of very low effectiveness.

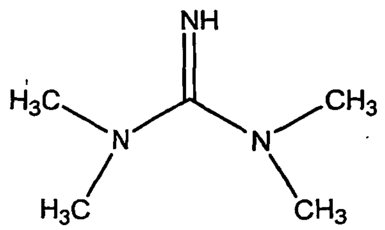

(IX)

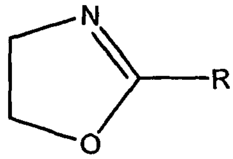

(X)

Thus it appears that for good antioxidant effectiveness the desired structural feature is that of a cyclic carboxamidine, where the imine group and an amine group are attached to the same carbon atom of a 5-membered or a 6-membered heterocyclic ring, thus forming either an imidazoline of structure (III) or a tetrahydropyrimidine of structure (V).

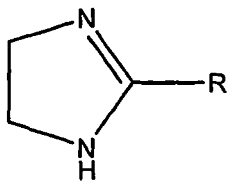

(III)

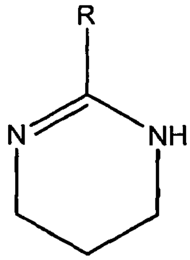

(V)

The methods for preparing imidazolines and tetrahydropyrimidines are simple and well known in the art and have been reported in the patent literature since the 1930's (cf. US patent 2,155,877). However, particularly in the cases where the starting amine is of the polyamine type, the published preparation procedures, despite their simplicity, require heating of the reactants at temperatures up to 300° C for long periods of time. It was found that addition to the reaction mixture of a small amount of a strong organic base (e.g. tetramethylguanidine) can speed up the reaction and permit its completion at lower temperatures.

Having described the invention in general terms, we will proceed with specific examples that illustrate its implementation and efficacy.

SPECIFIC EXAMPLES

Examples 1 to 10 were pure chemical entities, produced by the reaction of a single carboxylic moiety of formula (VI) with either 1,2-diaminoethane (II) or 1,3-diaminopropane (IV); thus their chemical structure was that of either a substituted imidazoline (III) or a substituted tetrahydropyrimidine (V). Comparison examples 11 and 12 had the oxazoline structure (X), whereas comparison example 13 was tetramethylguanidine (IX), obtained from a chemical supply house. Experimental details are provided for the preparation of examples 1, 5, and 11 which are typical for the three classes of compounds (III), (V), and (X). Table 1 lists details of all the above examples.

Examples 14 to 30 were prepared from mixtures of carboxylic moieties of formula (VI), mixtures of amine moieties of formula (VII), or both. In these cases it was found advantageous to add a small amount of tetramethylguanidine (IX) as a reaction promoter. Experimental details are provided for the preparation of Examples 14 and 28, which is typical of all the others. Table 2 lists details of examples 14 to 30.

Example 31 demonstrates the effectiveness of examples 1 to 30 as antioxidants in biodiesel, based on experimental measurements. Example 32 deals with the antioxidant effectiveness in blends of biodiesel with conventional petrodiesel, as required by the latest European Standard EN 590. Finally, example 33 addresses the effect on acid number of representative examples.

EXAMPLE 1

In a round bottom flask equipped with a water separator and a reflux condenser were placed 50 g (0.25 mol) of lauric acid dissolved in 150 mL toluene and then 16.8 g (0.28 mol) of 1,2-diaminoethane were added dropwise and with stirring. The mixture was heated under reflux until water stopped separating and then the toluene was distilled off

*in vacuo.* The solid residue was recrystallized from a mixture of toluene and heptane, thus affording the desired 2-undecyl imidazoline as colorless crystals, m.p. 123-125° C.

EXAMPLE 5

Lauric acid and 1,3-diaminopropane were reacted in the same fashion as example 1 and afforded 2-undecyl tetrahydropyrimidine, m.p. 66-67° C.

COMPARISON EXAMPLE 11

Lauric acid and 2-aminoethanol were reacted in the same fashion as example 1 and afforded 2-undecyl oxazoline, m.p. 71-74° C.

TABLE 1

| ENTITY | STRUCTURE | R |
|---|---|---|
| EXAMPLE 1 | (III) | n-Undecyl |
| EXAMPLE 2 | (III) | n-Pentadecyl |
| EXAMPLE 3 | (V) | n-Butyl |
| EXAMPLE 4 | (V) | n-Heptyl |
| EXAMPLE 5 | (V) | n-Undecyl |
| EXAMPLE 6 | (V) | n-Tridecyl |
| EXAMPLE 7 | (V) | n-Pentadecyl |
| EXAMPLE 8 | (V) | n-Heptadecyl |
| EXAMPLE 9 | (V) | Cyclohexyl |
| EXAMPLE 10 | (V) | Oleyl |
| COMPARISON EXAMPLE 11 | (X) | n-Undecyl |
| COMPARISON EXAMPLE 12 | (X) | Oleyl |
| COMPARISON EXAMPLE 13 | (IX) | - |

EXAMPLE 14

50 g of commercial tetraethylenepentamine (TEPA) was placed in a 500 mL round bottom flask and 0.25 g of tetramethylguanidine was added. The mixture was brought to a temperature of 100° C with stirring under a nitrogen atmosphere. Technical oleic acid of 90% purity (141 g) was then added dropwise over a period of 30 minutes with continued stirring. A vacuum of about 50 mm Hg was then applied and the temperature was increased to 170° C and was kept there for 4 hours. The heating was then discontinued, the vacuum was broken with nitrogen being admitted, and the flask was allowed to cool to room temperature. The product was a light brown viscous oil and its weight revealed the loss of about 1 mol of water.

EXAMPLE 28

The procedure of example 14 was repeated, except that the oleic acid was replaced by 130 g of a mixture of fatty acids obtained from the saponification of cottonseed oil. The product was a viscous and clear brown oil.

TABLE 2

| ENTITY | CARBOXYLIC MOIETY | AMINE MOIETY |
|---|---|---|
| EXAMPLE 14 | OLEIC ACID | TEPA |
| EXAMPLE 15 | BENZOIC ACID | DUOMEEN C |
| EXAMPLE 16 | OCTANOIC ACID | DUOMEEN C |

(continued)

| ENTITY | CARBOXYLIC MOIETY | AMINE MOIETY |
|---|---|---|
| EXAMPLE 17 | VALERIC ACID | DUOMEEN C |
| EXAMPLE 18 | CYCLOHEXANOIC ACID | DUOMEEN C |
| EXAMPLE 19 | LAURIC ACID | DUOMEEN C |
| EXAMPLE 20 | OLEIC ACID | DUOMEEN C |
| EXAMPLE 21 | LAURIC ACID | DUOMEEN O |
| EXAMPLE 22 | LAURIC ACID | DETA |
| EXAMPLE 23 | CYCLOHEXANOIC ACID | DETA |
| EXAMPLE 24 | METHYL OLEATE | TEPA |
| EXAMPLE 25 | METHYL OLEATE | TETA |
| EXAMPLE 26 | LAURIC ACID | TETA |
| EXAMPLE 27 | FFA 1 | DETA |
| EXAMPLE 28 | FFA 1 | TEPA |
| EXAMPLE 29 | FFA 2 | DETA |
| EXAMPLE 30 | FFA 2 | TEPA |

Because of space limitations in Table 2 certain abbreviations are used, whose meaning is the following:

- DETA, TETA, and TEPA are common names for the commercially available mixtures of ethylene polyamines, also known as diethylene triamine, triethylene tetramine, and tetraethylene pentamine respectively.
- DUOMEEN C is the commercial name of a mixture of 1-alkyl-1,3-propanediamines, whose alkyl groups are ultimately derived from coconut oil via an intermediate coco-amine. DUOMEEN O is a similar mixture, ultimately derived from olive oil via an intermediate oleylamine.
- FFA 1 is a mixture of free fatty acids that was produced by saponification of cottonseed oil. FFA 2 was similarly derived from rapeseed oil.

EXAMPLE 31

To test the antioxidant activity of the above additives, a biodiesel base fuel was utilized and was designated as Biodiesel A. It was prepared by base catalyzed transesterification with methanol of a vegetable oil blend consisting of 65% rapeseed oil and 35% used frying oil. Two more biodiesel base fuels were used in some of the experiments, designated as Biodiesel B and Biodiesel C; the former was made from a mixture of 36% used frying oil, 20% palm oil, and 44% soybean oil and the latter from a mixture of 32.4% used frying oil, 36% rapeseed oil, and 21.6% soybean oil. Some of the important characteristics of these three biodiesel base fuels are shown in Table 3, along with the respective limits in the European Standard EN 14214 and the methods that were used to measure them.

TABLE 3

| Property | Methyl Ester Content | Sulfur Content | Water Content | Acid Number | Oxidation Stability |
|---|---|---|---|---|---|
| Unit | % m/m | mg/kg | mg/kg | mg KOH/g | hours |
| Biodiesel A | 96.7 | 5.0 | 352 | 0.47 | 4.8 |
| Biodiesel B | 97.8 | 5.0 | 356 | 0.28 | 4.15 |
| Biodiesel C | 97.9 | 5.0 | 300 | 0.26 | 6.5 |
| Limit | 96.5 min | 10 max | 500 max | 0.50 max | 6 min |
| Method | EN 14103 | EN ISO 20846 | EN ISO 12937 | EN 14104 | EN 14112 |

The method that was used to measure the oxidation stability in all cases was EN 14112, which is prescribed

in the European Standard EN 14214 and is commonly known as the Rancimat test. It measures, under specified conditions, the induction period before the onset of rapid oxidation; the minimum acceptable value is 6 hours according to EN 14214.

Each of the examples 1-10 and 14-30 was separately added to Biodiesel A at a concentration of 1000 ppm (0.1% m/m) and the oxidation stability was measured by the Rancimat test in triplicate. For comparison purposes the same procedure was used with comparison examples 11-13 and also with BHT, one of the most widely used phenolic antioxidant additives for biodiesel. To make comparisons easier, the relative effectiveness of each example in comparison to BHT was computed according to formula (3):

$$R = (S_E - S_0) / (S_B - S_0) \qquad (3)$$

Where R is the relative effectiveness in comparison to BHT, $S_E$ is the induction period of the biodiesel containing the example additive, So is the induction period of the neat biodiesel, and $S_B$ is the induction period of the biodiesel containing BHT.

Table 4 shows the Rancimat induction period (average of 3 determinations) and the relative performance of all examples.

TABLE 4

| ADDITIVE | INDUCTION PERIOD (hours) | RELATIVE EFFECTIVENESS |
|---|---|---|
| NONE | 4.80 | - |
| BHT | 8.89 | 1.00 |
| EXAMPLE 1 | 5.91 | 0.27 |
| EXAMPLE 2 | 6.22 | 0.35 |
| EXAMPLE 3 | 10.45 | 1.38 |
| EXAMPLE 4 | 8.94 | 1.01 |
| EXAMPLE 5 | 11.64 | 1.67 |
| EXAMPLE 6 | 9.43 | 1.13 |
| EXAMPLE 7 | 8.99 | 1.02 |
| EXAMPLE 8 | 8.29 | 0.85 |
| EXAMPLE 9 | 9.67 | 1.19 |
| EXAMPLE 10 | 9.72 | 1.20 |
| EXAMPLE 14 | 12.06 | 1.78 |
| EXAMPLE 15 | 11.48 | 1.63 |
| EXAMPLE 16 | 10.69 | 1.44 |
| EXAMPLE 17 | 10.31 | 1.35 |
| EXAMPLE 18 | 9.55 | 1.16 |
| EXAMPLE 19 | 9.08 | 1.05 |
| EXAMPLE 20 | 8.89 | 1.00 |
| EXAMPLE 21 | 8.88 | 1.00 |
| EXAMPLE 22 | 8.91 | 1.00 |
| EXAMPLE 23 | 7.58 | 0.68 |
| EXAMPLE 24 | 10.28 | 1.34 |
| EXAMPLE 25 | 8.91 | 1.00 |
| EXAMPLE 26 | 7.69 | 0.71 |

(continued)

| ADDITIVE | INDUCTION PERIOD (hours) | RELATIVE EFFECTIVENESS |
|---|---|---|
| EXAMPLE 27 | 11.24 | 1.57 |
| EXAMPLE 28 | 14.65 | 2.41 |
| EXAMPLE 29 | 12.52 | 1.89 |
| EXAMPLE 30 | 15.89 | 2.71 |
| COMPARISON EXAMPLE 11 | 5.17 | 0.09 |
| COMPARISON EXAMPLE 12 | 5.02 | 0.05 |
| COMPARISON EXAMPLE 13 | 4.79 | 0.00 |

On examining the results shown in Table 4, the following observations can be made:

- All 27 examples of this invention improved the oxidative stability of the biodiesel base fuel.
- The oxidation stability improvement in all but one of the examples was enough to bring the off-specification base biodiesel A to within specification.
- A large majority of the examples (20 out of 27) were more effective than BHT in improving the oxidative stability of biodiesel A, with two of them being more than twice as effective.
- Comparison examples 11 and 12 (which possessed the oxazoline moiety) showed only a very slight antioxidant activity, whereas the acyclic amidine of comparison example 13 displayed a complete lack of such activity.

Several of the more effective examples in Table 4 were also tested in the more prone to oxidation Biodiesel B and at a lower concentration of 500 ppm (0.05% m/m). The results of the Rancimat test are shown in Table 5.

TABLE 5

| ADDITIVE | INDUCTION PERIOD (hours) | RELATIVE EFFECTIVENESS |
|---|---|---|
| NONE | 4.15 | - |
| BHT | 6.65 | 1.00 |
| EXAMPLE 3 | 8.39 | 1.70 |
| EXAMPLE 5 | 8.04 | 1.56 |
| EXAMPLE 9 | 7.74 | 1.44 |
| EXAMPLE 14 | 7.99 | 1.54 |
| EXAMPLE 15 | 8.30 | 1.66 |
| EXAMPLE 16 | 8.08 | 1.57 |
| EXAMPLE 17 | 7.52 | 1.35 |
| EXAMPLE 24 | 7.80 | 1.46 |
| EXAMPLE 27 | 8.44 | 1.72 |
| EXAMPLE 28 | 10.12 | 2.39 |
| EXAMPLE 29 | 9.30 | 2.06 |
| EXAMPLE 30 | 10.54 | 2.56 |

All the examples listed in Table 5 showed high antioxidant activity at the lower concentration of 500 ppm and they were all significantly more effective than the phenolic antioxidant BHT in this respect.

EXAMPLE 32

Given that the newest European Standard for road diesel EN 590 has a requirement for minimum oxidation stability of the final biodiesel/petrodiesel blend, a series of experiments were run to test the efficacy of the additives of this invention in this respect. Biodiesel C was blended in several concentrations with an ultra low sulfur conventional diesel fuel meeting the current European specifications. Example 29 was used as the antioxidant additive and was compared with the well known phenolic antioxidant TBHQ.

Three batches of Biodiesel C were used for this set of experiments. One was neat, the second contained 1000 ppm of the additive of example 29, and the third contained 1000 ppm of TBHQ. Neat Biodiesel C had an oxidation stability of 6.5 hours, as already mentioned in Table 3; the stability of the second batch was increased to 12.5 hours by the presence of Example 29, whereas the stability of the third batch was brought up to an impressive 21.6 hours by TBHQ, which thus appeared as an extremely effective antioxidant additive for biodiesel.

The three batches were mixed at various proportions with petrodiesel and the oxidation stability of the resulting blends was measured by the modified Rancimat method (EN 15751) as required by EN 590. The specification limit for the induction period in blends is set as 20 hours minimum.

Table 6 contains the results of these tests.

TABLE 6

| BIODIESEL IN THE BLEND | INDUCTION TIME (hours) | | |
|---|---|---|---|
| | NO ANTIOXIDANT | EXAMPLE 29 | TBHQ |
| 100% | 6.50 | 12.50 | 21.60 |
| 10% v/v | 22.68 | 32.61 | 24.05 |
| 7% v/v | 28.66 | 35.43 | 27.48 |
| 5% v/v | 31.66 | 37.79 | 28.70 |
| 4% v/v | 33.22 | 39.58 | 29.81 |
| 3% v/v | 34.48 | 39.55 | 30.80 |
| 2% v/v | 37.74 | 38.34 | 32.57 |

The results in Table 6 show that Example 29 of this invention is effective in enhancing the oxidative stability of biodiesel whether it is neat or blended with conventional diesel fuel in various proportions. The situation is completely different with the phenolic antioxidant TBHQ; it is extremely effective in stabilizing neat biodiesel but in the case of blends its effectiveness disappears and it actually behaves as a pro-oxidant when the biodiesel concentration is 7% or less.

EXAMPLE 33

To measure the effect of additives on biodiesel acidity several samples were prepared, each one being a solution containing 1000 ppm of either a phenolic antioxidant or one of the examples of the present invention in one of the biodiesel base fuels A, B, or C. The acid number of each sample was measured with standard method EN 14104 and the results are shown in Table 7.

All phenolic antioxidants increase the acid number of the biodiesel they are dissolved in, which is expected on account of the acidity of the phenolic hydroxyls that they all contain. By contrast, all examples of the present invention reduced the acid number of the biodiesel, a fact that is attributed to the basicity of their chemical structures which can all be classed as amine derivatives.

TABLE 7

| ADDITIVE | ACID NUMBER (mg KOH/g) | | |
|---|---|---|---|
| | Biodiesel A | Biodiesel B | Biodiesel C |
| NONE | 0.47 | 0.28 | 0.26 |
| BHT* | 0.53 | 0.51 | 0.29 |
| TBHQ* | 0.49 | 0.44 | 0.32 |

(continued)

| ADDITIVE | ACID NUMBER (mg KOH/g) | | |
|---|---|---|---|
| | Biodiesel A | Biodiesel B | Biodiesel C |
| PROPYL GALLATE* | 0.55 | 0.52 | 0.38 |
| EXAMPLE 3 | 0.35 | 0.26 | 0.24 |
| EXAMPLE 5 | 0.28 | 0.21 | 0.20 |
| EXAMPLE 14 | 0.34 | 0.23 | 0.21 |
| EXAMPLE 27 | 0.29 | 0.24 | 0.23 |
| EXAMPLE 29 | 0.27 | 0.24 | 0.22 |
| * phenolic antioxidant | | | |

**Claims**

1. Use of a substance or a mixture of substances as an additive for improving oxidation stability and lowering acid number of a biodiesel composition, wherein said substance or mixture of substances is the product of the chemical reaction between Component 1 and Component 2, wherein:

   Component 1 is a carboxylic acid, a carboxylic ester or a mixture of carboxylic acids and/or esters having the chemical formula:

   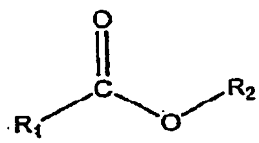

   (VI)

   where:

   $R_1$ is a straight or branched alkyl or hydroxyalkyl group having the formula $C_mH_{2m-x-k+1}(OH)_k$ (m being an integer from 1 to 30, x being O, 2, 4 or 6 but always fulfilling the relation x < m, and k being O or 1), a cyclohexyl group, a phenyl group, or a substituted phenyl group bearing a $C_1$ to $C_{12}$ alkyl substituent, and $R_2$ is hydrogen, methyl, or ethyl.

   Component 2 is a non-aromatic diamine or polyamine having at least one primary nitrogen atom separated by two or three carbon atoms from another nitrogen atom which is primary or secondary, or a mixture of such diamines and/or polyamines having the chemical formula:

   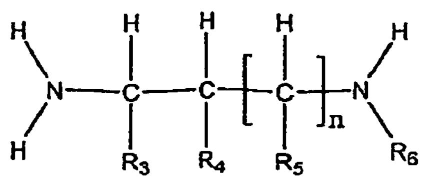

   (VII)

   where:

   n is 0 or 1, $R_3$ and $R_4$ are hydrogen, methyl, or taken together are part of a cyclohexane ring, $R_5$ is hydrogen, or methyl, and $R_6$ is hydrogen, a straight or branched alkyl group having the formula $C_mH_{2m-x+1}$ (m being an integer from 1 to 22 and x being 0, 2, 4, or 6 but always fulfilling the relation x < m), or an amine moiety having the formula $-CH_2CH_2(CH_2)_pNH[CH_2CH_2(CH_2)_pNH]_qH$ with p being 0 or 1 and q being 0, 1, 2, 3, or 4.

2. Use according to claim 1, where Component 2 is N-(2-aminoethyl-1,2-ethanediamine), also known as Diethylene

Triamine or DETA.

**3.** Use according to claim 1, where Component 2 is the mixed polyamine Triethylene Tetramine or TETA.

**4.** Use according to claim 1, where Component 2 is the mixed polyamine Tetraethylene Pentamine or TEPA.

**5.** Use according to claim 1, where Component 1 is a mixture of fatty acids obtained by the saponification of a vegetable or animal oil or fat or a mixture of such oils or fats.

**6.** Use according to claim 1, where the substance or mixture of substances is present at a concentration between 0.01 percent by weight and 1 percent by weight, based on the total amount of the biodiesel composition.

**7.** Use according to claim 1, where the additive is employed in the form of an additive concentrate composition comprising:

a. at least 5 percent by weight of a substance or mixture of substances which is the product of the chemical reaction between Component 1 and Component 2 according to claim 1, and
b. up to 95 percent by weight of a diluent consisting of a liquid mixture of hydrocarbons having a flash point of at least 100°C and a final boiling point of less than 340°C, or of a biodiesel fuel suitable for use in a compression ignition engine and consisting of a mixture of methyl or ethyl esters of fatty acids derived from fats and oils of vegetable or animal origin, or of a blend of liquid hydrocarbons and biodiesel fuel as defined hereinabove.

**8.** Use according to claim 1, where the biodiesel composition is a component of a diesel fuel composition comprising:

• Conventional diesel fuel derived from petroleum or other mineral fuels and suitable for use in a compression ignition engine, to the extent of 99 percent or less by volume, and
• a biodiesel composition according to claim 1, to the extent of one percent or more by volume.

**9.** A biodiesel composition containing non-phenolic additives to improve oxidation stability and to lower acid number, comprising:

a. a major amount of biodiesel fuel suitable for use in a compression ignition engine and consisting of a mixture of methyl or ethyl esters of fatty acids derived from fats and oils of vegetable or animal origin, and
b. a minor amount of a substance or mixture of substances which is the product of the chemical reaction between Component 1 and Component 2, said product having the structural feature of a cyclic carboxamidine, where the imine group and an amine group are attached to the same carbon atom of a 5-membered or a 6-membered heterocyclic ring, thus forming either an imidazoline of structure (III) or a tetrahydropyrimidine of structure (V).

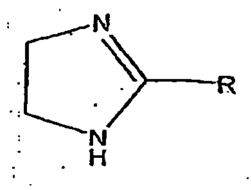

(III)

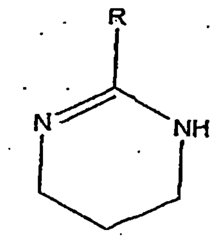

(V)

wherein:

Component 1 is a carboxylic acid, a carboxylic ester or a mixture of carboxylic acids and/or esters having the chemical formula:

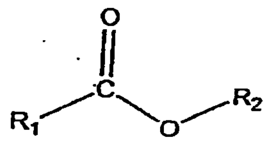

(VI)

where:

$R_1$ is a straight or branched alkyl or hydroxyalkyl group having the formula $C_mH_{2m-x-k+1}(OH)_k$ (m being an integer from 1 to 30, x being 0, 2, 4, or 6 but always fulfilling the relation x<m, and k being O or 1), a cyclohexyl group, a phenol group, or a substituted phenyl group bearing a $C_1$ to $C_{12}$ alkyl substituent, and $R_2$ is hydrogen, methyl, or ethyl.

Component 2 is a non-aromatic diamine having at least one primary nitrogen atom separated by two or three carbon atoms from another nitrogen atom which is primary, or a mixture of such diamines having the chemical formula:

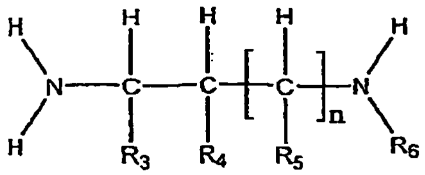

(VII)

where:

n is 0 or 1, $R_3$ and $R_4$ are hydrogen, methyl, or taken together are part of a cyclohexane ring, $R_5$ is hydrogen, or methyl, and $R_6$ is a straight or branched alkyl group having the formula $C_mH_{2m-x+1}$ (m being an integer from 1 to 22 and x being 0,2,4 or 6 but always fulfilling the relation x<m)

**10.** A biodiesel composition according to claim 9, where Component 1 is a mixture of fatty acids obtained by the saponification of a vegetable or animal oil or fat or a mixture of such oils or fats.

**11.** A biodiesel composition according to Claim 9, where the substance or mixture of substances described in paragraph b. is present at a concentration between 0.01 percent by weight and 1 percent by weight.

**12.** A biodiesel composition according to claim 9, which also contains an appropriate amount of additives for improving some of its properties such as ignition quality, cold flow characteristics, resistance to microbial degradation, tribological characteristics, and the like.

**13.** An additive concentrate composition comprising:

a. at least 5 percent by weight of a substance or mixture of substances which is the product of the chemical reaction between Component 1 and Component 2 as defined in Claim 9, and
b. up to 95 percent by weight of a diluent consisting of a liquid mixture of hydrocarbons having a flash point of at least 100° C and a final boiling point of less than 340° C, or of a biodiesel fuel suitable for use in a compression ignition engine and consisting of a mixture of methyl or ethyl esters of fatty acids derived from fats and oils of vegetable or animal origin, or of a blend of liquid hydrocarbons and biodiesel fuel as defined hereinabove.

**14.** A diesel fuel composition comprising:

• Conventional diesel fuel derived from petroleum or other mineral fuels and suitable for use in a compression ignition engine, to the extent of 99 percent or less by volume, and
• A biodiesel composition according to Claim 9, to the extent of one percent or more by volume.

**15.** A diesel fuel composition according to claim 9, which also contains an appropriate amount of additives for improving

some of its properties such as ignition quality, cold flow characteristics, resistance to microbial degradation, tribological characteristics, and the like.

## Patentansprüche

**1.** Verwendung einer Substanz oder eines Gemischs von Substanzen als Additiv zur Verbesserung der Oxidationsstabilität und Senkung der Säurezahl einer Biodieselzusammensetzung, wobei es sich bei der Substanz oder dem Gemisch von Substanzen um das Produkt der chemischen Reaktion zwischen Komponente 1 und Komponente 2 handelt, wobei:

Komponente 1 eine Carbonsäure, ein Carbonsäureester oder ein Gemisch von Carbonsäuren und/oder Carbonsäureestern mit der chemischen Formel:

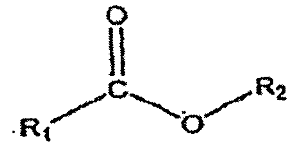

(VI)

ist, wobei:

$R_1$ für eine gerade oder verzweigte Alkyl- oder Hydroxyalkylgruppe mit der Formel $C_mH_{2m-x-k+1}(OH)_k$ (wobei m für eine ganze Zahl von 1 bis 30 steht, x für 0, 2, 4 oder 6 steht, aber immer die Beziehung $x < m$ erfüllt, und k für 0 oder 1 steht), eine Cyclohexylgruppe, eine Phenylgruppe oder eine substituierte Phenylgruppe mit einem $C_1$- bis $C_{12}$-Alkylsubstituenten steht und $R_2$ für Wasserstoff, Methyl oder Ethyl steht;

Komponente 2 ein nichtaromatisches Diamin oder Polyamin mit mindestens einem primären Stickstoffatom, das durch zwei oder drei Kohlenstoffatome von einem weiteren Stickstoffatom, das primär oder sekundär ist, getrennt ist, oder ein Gemisch derartiger Diamine und/oder Polyamine mit der chemischen Formel:

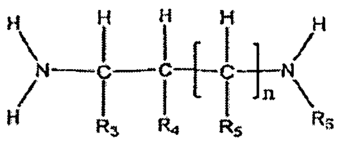

(VII)

ist, wobei:

n für 0 oder 1 steht, $R_3$ und $R_4$ für Wasserstoff oder Methyl stehen oder zusammengenommen Teil eines Cyclohexanrings sind, $R_5$ für Wasserstoff oder Methyl steht und $R_6$ für Wasserstoff, eine gerade oder verzweigte Alkylgruppe mit der Formel $C_mH_{2m-x+1}$ (wobei m für eine ganze Zahl von 1 bis 22 steht und x für 0, 2, 4 oder 6 steht, aber immer die Beziehung $x < m$ erfüllt) oder eine Amingruppierung mit der Formel $-CH_2CH_2(CH_2)_pNH[CH_2CH_2(CH_2)_pNH]_qH$ steht, wobei p für 0 oder 1 steht und q für 0, 1, 2, 3 oder 4 steht.

**2.** Verfahren nach Anspruch 1, wobei es sich bei Komponente 2 um N-(2-Aminoethyl-1,2-ethandiamin), das auch als Diethylentriamin oder DETA bekannt ist, handelt.

**3.** Verwendung nach Anspruch 1, wobei es sich bei Komponente 2 um das gemischte Polyamin Triethylentetramin oder TETA handelt.

**4.** Verwendung nach Anspruch 1, wobei es sich bei Komponente 2 um das gemischte Polyamin Tetraethylenpentamin oder TEPA handelt.

**5.** Verwendung nach Anspruch 1, wobei es sich bei Komponente 1 um ein durch die Verseifung eines pflanzlichen oder tierischen Öls oder Fetts oder eines Gemischs derartiger Öle oder Fette erhaltenes Gemisch von Fettsäuren handelt.

**6.** Verwendung nach Anspruch 1, wobei die Substanz bzw. das Gemisch von Substanzen in einer Konzentration zwischen 0,01 Gewichtsprozent und 1 Gewichtsprozent, bezogen auf die Gesamtmenge der Biodieselzusammensetzung, vorliegt.

**7.** Verwendung nach Anspruch 1, wobei das Additiv in Form einer Additivkonzentratzusammensetzung, umfassend:

a. mindestens 5 Gewichtsprozent einer Substanz oder eines Gemischs von Substanzen, bei der bzw. dem es sich um das Produkt der chemischen Reaktion zwischen Komponente 1 und Komponente 2 gemäß Anspruch 1 handelt, und
b. bis zu 95 Gewichtsprozent eines Verdünnungsmittels, das aus einem flüssigen Gemisch von Kohlenwasserstoffen mit einem Flammpunkt von mindestens 100°C und einem Siedeende von weniger als 340°C oder aus einem Biodieselkraftstoff, der zur Verwendung in einem Kompressionszündungsmotor geeignet ist und aus einem Gemisch von Methyl- oder Ethylestern von Fettsäuren, die sich von Fetten und Ölen pflanzlichen oder tierischen Ursprungs ableiten, oder aus einer Mischung von flüssigen Kohlenwasserstoffen und Biodieselkraftstoff gemäß obiger Definition besteht, eingesetzt wird.

**8.** Verwendung nach Anspruch 1, wobei es sich bei der Biodieselzusammensetzung um eine Komponente einer Dieselkraftstoffzusammensetzung, umfassend:

• herkömmlichen Dieselkraftstoff, der sich von Erdöl oder anderen mineralischen Kraftstoffen ableitet und zur Verwendung in einem Kompressionszündungsmotor geeignet ist, in einer Menge von 99 Volumenprozent oder weniger und
• eine Biodieselzusammensetzung nach Anspruch 1 in einer Menge von einem Volumenprozent oder mehr, handelt.

**9.** Biodieselzusammensetzung mit nichtphenolischen Additiven zur Verbesserung der Oxidationsstabilität und Senkung der Säurezahl, umfassend:

a. eine größere Menge von Biodieselkraftstoff, der zur Verwendung in einem Kompressionszündungsmotor geeignet ist und aus einem Gemisch von Methyl- oder Ethylestern von Fettsäuren, die sich von Fetten und Ölen pflanzlichen oder tierischen Ursprungs ableiten, besteht, und
b. eine kleinere Menge einer Substanz bzw. eines Gemischs von Substanzen, bei der bzw. dem es sich um das Produkt der chemischen Reaktion zwischen Komponente 1 und Komponente 2 handelt, wobei das Produkt das Strukturmerkmal eines cyclischen Carboxamidins aufweist, wobei die Imingruppe und eine Amingruppe an dasselbe Kohlenstoffatom eines 5-gliedrigen oder 6-gliedrigen heterocyclischen Rings gebunden sind, wodurch entweder ein Imidazolin der Struktur (III) oder ein Tetrahydropyrimidin der Struktur (V) gebildet wird:

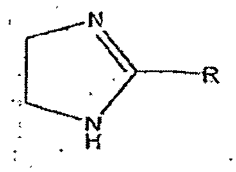
(III)

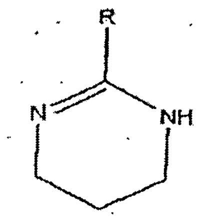
(V)

wobei:

Komponente 1 eine Carbonsäure, ein Carbonsäureester oder ein Gemisch von Carbonsäuren und/oder Carbonsäureestern mit der chemischen Formel:

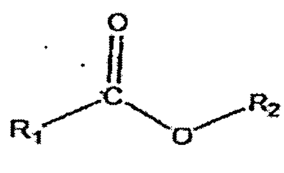
(VI)

ist, wobei:

$R_1$ für eine gerade oder verzweigte Alkyl- oder Hydroxyalkylgruppe mit der Formel $C_mH_{2m-x-k+1}(OH)_k$ (wobei m für eine ganze Zahl von 1 bis 30 steht, x für 0, 2, 4 oder 6 steht, aber immer die Beziehung x < m erfüllt, und k für 0 oder 1 steht), eine Cyclohexylgruppe, eine Phenylgruppe oder eine substituierte Phenylgruppe mit einem $C_1$- bis $C_{12}$-Alkylsubstituenten steht und $R_2$ für Wasserstoff, Methyl oder Ethyl steht;

Komponente 2 ein nichtaromatisches Diamin mit mindestens einem primären Stickstoffatom, das durch zwei oder drei Kohlenstoffatome von einem weiteren Stickstoffatom, das primär ist, getrennt ist, oder ein Gemisch derartiger Diamine mit der chemischen Formel:

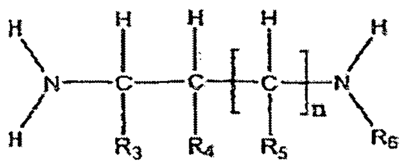

(VII)

ist, wobei:

n für 0 oder 1 steht, $R_3$ und $R_4$ für Wasserstoff oder Methyl stehen oder zusammengenommen Teil eines Cyclohexanrings sind, $R_5$ für Wasserstoff oder Methyl steht und $R_6$ für eine gerade oder verzweigte Alkylgruppe mit der Formel $C_mH_{2m-x+1}$ (wobei m für eine ganze Zahl von 1 bis 22 steht und x für 0, 2, 4 oder 6 steht, aber immer die Beziehung x < m erfüllt) steht.

**10.** Biodieselzusammensetzung nach Anspruch 9, wobei es sich bei Komponente 1 um ein durch die Verseifung eines pflanzlichen oder tierischen Öls oder Fetts oder eines Gemischs derartiger Öle oder Fette erhaltenes Gemisch von Fettsäuren handelt.

**11.** Biodieselzusammensetzung nach Anspruch 9, wobei die Substanz bzw. das Gemisch von Substanzen gemäß Absatz b. in einer Konzentration zwischen 0,01 Gewichtsprozent und 1 Gewichtsprozent vorliegt.

**12.** Biodieselzusammensetzung nach Anspruch 9, die außerdem eine geeignete Menge von Additiven zur Verbesserung einiger ihrer Eigenschaften wie Zündqualität, Kaltfließeigenschaften, Beständigkeit gegenüber mikrobiellem Abbau, tribologischen Eigenschaften und dergleichen enthält.

**13.** Additivkonzentratzusammensetzung, umfassend:

a. mindestens 5 Gewichtsprozent einer Substanz oder eines Gemischs von Substanzen, bei der bzw. dem es sich um das Produkt der chemischen Reaktion zwischen Komponente 1 und Komponente 2 gemäß Anspruch 9 handelt, und
b. bis zu 95 Gewichtsprozent eines Verdünnungsmittels, das aus einem flüssigen Gemisch von Kohlenwasserstoffen mit einem Flammpunkt von mindestens 100°C und einem Siedeende von weniger als 340°C oder aus einem Biodieselkraftstoff, der zur Verwendung in einem Kompressionszündungsmotor geeignet ist und aus einem Gemisch von Methyl- oder Ethylestern von Fettsäuren, die sich von Fetten und Ölen pflanzlichen oder tierischen Ursprungs ableiten, oder aus einer Mischung von flüssigen Kohlenwasserstoffen und Biodieselkraftstoff gemäß obiger Definition besteht.

**14.** Dieselkraftstoffzusammensetzung, umfassend:

- herkömmlichen Dieselkraftstoff, der sich von Erdöl oder anderen mineralischen Kraftstoffen ableitet und zur Verwendung in einem Kompressionszündungsmotor geeignet ist, in einer Menge von 99 Volumenprozent oder weniger und
- eine Biodieselzusammensetzung nach Anspruch 9 in einer Menge von einem Volumenprozent oder mehr.

**15.** Dieselkraftstoffzusammensetzung nach Anspruch 9, die außerdem eine geeignete Menge von Additiven zur Verbesserung einiger ihrer Eigenschaften wie Zündqualität, Kaltfließeigenschaften, Beständigkeit gegenüber mikrobiellem Abbau, tribologischen Eigenschaften und dergleichen enthält.

## Revendications

1. Utilisation d'une substance ou d'un mélange de substances en tant qu'additif pour améliorer la stabilité à l'oxydation et abaisser l'indice d'acide d'une composition de biodiesel, dans laquelle ladite substance ou ledit mélange de substances est le produit de la réaction chimique entre le constituant 1 et le constituant 2, sachant que :

   le constituant 1 est un acide carboxylique, un ester carboxylique ou un mélange d'acides et/ou d'esters carboxyliques de formule chimique :

   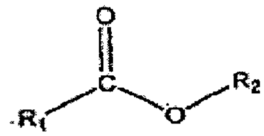

   (VI)

   dans laquelle :

   $R_1$ est un groupe alkyle ou hydroxyalkyle linéaire ou ramifié répondant à la formule $C_mH_{2m-x-k+1}(OH)_k$ (m étant un nombre entier de 1 à 30, x étant égal à 0, 2, 4 ou 6 mais satisfaisant toujours à la relation x < m, et k étant égal à 0 ou 1), un groupe cyclohexyle, un groupe phényle, ou un groupe phényle substitué portant un substituant alkyle en $C_1$ à $C_{12}$, et $R_2$ est un atome d'hydrogène ou un groupe méthyle ou éthyle ;

   le constituant 2 est une diamine ou polyamine non aromatique comportant au moins un atome d'azote primaire séparé par deux ou trois atomes de carbone d'un autre atome d'azote qui est primaire ou secondaire, ou un mélange de ces diamines et/ou polyamines de formule chimique :

   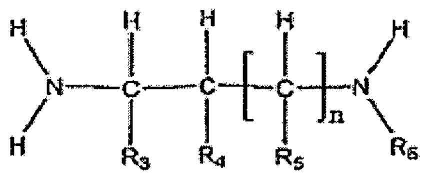

   (VII)

   dans laquelle :

   n est égal à 0 ou 1, $R_3$ et $R_4$ sont des atomes d'hydrogène ou des groupes méthyle, ou bien, pris conjointement, font partie d'un noyau cyclohexane, $R_5$ est un atome d'hydrogène ou un groupe méthyle, et $R_6$ est un atome d'hydrogène, un groupe alkyle linéaire ou ramifié répondant à la formule $C_mH_{2m-x+1}$ (m étant un nombre entier de 1 à 22 et x étant égal à 0, 2, 4 ou 6 mais satisfaisant toujours à la relation x < m), ou un groupement amine répondant à la formule $-CH_2CH_2(CH_2)_pNH[CH_2-CH_2(CH_2)_pNH]_qH$ où p est égal à 0 ou 1 et q est égal à 0, 1, 2, 3 ou 4.

2. Utilisation selon la revendication 1, dans laquelle le constituant 2 est la N-(2-aminoéthyl-1,2-éthanediamine), connue également sous le nom de diéthylènetriamine ou DETA.

3. Utilisation selon la revendication 1, dans laquelle le constituant 2 est la polyamine mixte triéthylènetétramine ou TETA.

4. Utilisation selon la revendication 1, dans laquelle le constituant 2 est la polyamine mixte tétraéthylènepentamine ou TEPA.

5. Utilisation selon la revendication 1, dans laquelle le constituant 1 est un mélange d'acides gras obtenus par saponification d'une huile ou graisse végétale ou animale ou d'un mélange de telles huiles ou graisses.

6. Utilisation selon la revendication 1, dans laquelle la substance ou le mélange de substances est présent à une

concentration comprise entre 0,01 pour cent en poids et 1 pour cent en poids, par rapport à la quantité totale de la composition de biodiesel.

**7.** Utilisation selon la revendication 1, dans laquelle l'additif est employé sous la forme d'une composition de concentré d'additif comprenant :

a. au moins 5 pour cent en poids d'une substance ou d'un mélange de substances qui est le produit de la réaction chimique entre le constituant 1 et le constituant 2 selon la revendication 1, et
b. jusqu'à 95 pour cent en poids d'un diluant constitué d'un mélange liquide d'hydrocarbures possédant un point d'éclair d'au moins 100°C et un point d'ébullition final inférieur à 340°C, ou d'un carburant biodiesel qui convient pour une utilisation dans un moteur à allumage par compression et qui est constitué d'un mélange d'esters méthyliques ou éthyliques d'acides gras dérivés de graisses et d'huiles d'origine végétale ou animale, ou d'un mélange d'hydrocarbures liquides et de carburant biodiesel tel que défini précédemment.

**8.** Utilisation selon la revendication 1, dans laquelle la composition de biodiesel est un constituant d'une composition de carburant diesel comprenant :

- un carburant diesel classique dérivé de pétrole ou d'autres combustibles minéraux et qui convient pour une utilisation dans un moteur à allumage par compression, à raison de 99 pour cent ou moins en volume, et
- une composition de biodiesel selon la revendication 1, à raison de un pour cent ou plus en volume.

**9.** Composition de biodiesel contenant des additifs non phénoliques pour améliorer la stabilité à l'oxydation et abaisser l'indice d'acide, comprenant :

a. une quantité majoritaire de carburant biodiesel qui convient pour une utilisation dans un moteur à allumage par compression et qui est constitué d'un mélange d'esters méthyliques ou éthyliques d'acides gras dérivés de graisses et d'huiles d'origine végétale ou animale, et
b. une quantité minoritaire d'une substance ou d'un mélange de substances qui est le produit de la réaction chimique entre le constituant 1 et le constituant 2, ledit produit comportant la caractéristique structurale d'une carboxamidine cyclique, dans laquelle le groupe imine et un groupe amine sont fixés sur le même atome de carbone d'un noyau hétérocyclique à 5 chaînons ou à 6 chaînons, formant ainsi soit une imidazoline de structure (III), soit une tétrahydropyrimidine de structure (V)

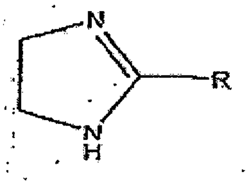
(III)

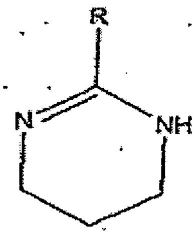
(V)

composition dans laquelle :

le constituant 1 est un acide carboxylique, un ester carboxylique ou un mélange d'acides et/ou d'esters carboxyliques de formule chimique :

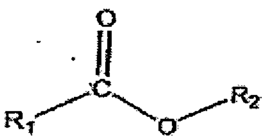
(VI)

dans laquelle :

$R_1$ est un groupe alkyle ou hydroxyalkyle linéaire ou ramifié répondant à la formule $C_mH_{2m-x-k+1}(OH)_k$

(m étant un nombre entier de 1 à 30, x étant égal à 0, 2, 4 ou 6 mais satisfaisant toujours à la relation x < m, et k étant égal à 0 ou 1), un groupe cyclohexyle, un groupe phényle, ou un groupe phényle substitué portant un substituant alkyle en $C_1$ à $C_{12}$, et $R_2$ est un atome d'hydrogène ou un groupe méthyle ou éthyle ;

le constituant 2 est une diamine non aromatique comportant au moins un atome d'azote primaire séparé par deux ou trois atomes de carbone d'un autre atome d'azote qui est primaire, ou un mélange de ces diamines de formule chimique :

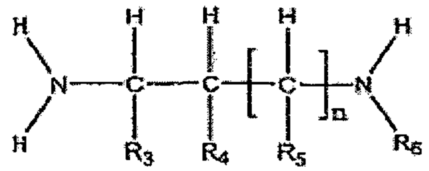

(VII)

dans laquelle :

n est égal à 0 ou 1, $R_3$ et $R_4$ sont des atomes d'hydrogène ou des groupes méthyle, ou bien, pris conjointement, font partie d'un noyau cyclohexane, $R_5$ est un atome d'hydrogène ou un groupe méthyle, et $R_6$ est un groupe alkyle linéaire ou ramifié répondant à la formule $C_mH_{2m-x+1}$ (m étant un nombre entier de 1 à 22 et x étant égal à 0, 2, 4 ou 6 mais satisfaisant toujours à la relation x < m).

**10.** Composition de biodiesel selon la revendication 9, dans laquelle le constituant 1 est un mélange d'acides gras obtenus par saponification d'une huile ou graisse végétale ou animale ou d'un mélange de telles huiles ou graisses.

**11.** Composition de biodiesel selon la revendication 9, dans laquelle la substance ou le mélange de substances décrit dans le paragraphe b. est présent à une concentration comprise entre 0,01 pour cent en poids et 1 pour cent en poids.

**12.** Composition de biodiesel selon la revendication 9, qui contient aussi une quantité appropriée d'additifs pour améliorer certaines de ses propriétés telles que la qualité de l'allumage, les caractéristiques d'écoulement à froid, la résistance à la dégradation microbienne, les caractéristiques tribologiques, et analogues.

**13.** Composition de concentré d'additif comprenant :

a. au moins 5 pour cent en poids d'une substance ou d'un mélange de substances qui est le produit de la réaction chimique entre le constituant 1 et le constituant 2 tels que définis dans la revendication 9, et
b. jusqu'à 95 pour cent en poids d'un diluant constitué d'un mélange liquide d'hydrocarbures possédant un point d'éclair d'au moins 100° C et un point d'ébullition final inférieur à 340° C, ou d'un carburant biodiesel qui convient pour une utilisation dans un moteur à allumage par compression et qui est constitué d'un mélange d'esters méthyliques ou éthyliques d'acides gras dérivés de graisses et d'huiles d'origine végétale ou animale, ou d'un mélange d'hydrocarbures liquides et de carburant biodiesel tel que défini précédemment.

**14.** Composition de carburant diesel comprenant :

- un carburant diesel classique dérivé de pétrole ou d'autres combustibles minéraux et qui convient pour une utilisation dans un moteur à allumage par compression, à raison de 99 pour cent ou moins en volume, et
- une composition de biodiesel selon la revendication 9, à raison de un pour cent ou plus en volume.

**15.** Composition de carburant diesel selon la revendication 9, qui contient aussi une quantité appropriée d'additifs pour améliorer certaines de ses propriétés telles que la qualité de l'allumage, les caractéristiques d'écoulement à froid, la résistance à la dégradation microbienne, les caractéristiques tribologiques, et analogues.

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- DE 10252714 A1
- DE 102005015474 A1
- EP 1989275 A
- WO 2008121526 A1
- WO 9419430 A
- WO 2008049822 A
- US 5470856 A
- US 5925665 A
- US 6294566 A
- US 6410562 A
- US 6875788 A
- US 6884801 A
- US 7173044 A
- US 7482358 A
- US 7057050 B
- US 4518782 B
- US 4247300 A
- US 2961308 A
- US 2155877 A

### Non-patent literature cited in the description

- **GERHARD KNOTHE.** Some aspects of biodiesel oxidative stability. *Fuel Processing Technology,* 2007, vol. 88, 669-677
- **SOUTHWEST RESEARCH INSTITUTE.** Characterization of biodiesel oxidation and oxidation products. *CRC Project No. AVFL-2b,* 2005
- **SIGURD SCHOBER ; MARTIN MITTELBACH.** The impact of antioxidants on biodiesel oxidation stability. *Eur. J. Lipid Sci. Technol.,* 2004, vol. 106, 382-389